# EUROPEAN PATENT SPECIFICATION

(11) **EP 4 255 915 B1**
(45) Date of publication and mention of the grant of the patent: **10.09.2025**
(21) Application number: 21823338.5
(22) Date of filing: 01.12.2021
(51) Int. Cl.: C11C 3/00, C11B 13/00, C07J 75/00, C07J 9/00

(54) **PROCESS FOR PURIFYING STEROLS FROM TALL OIL PITCH**
VERFAHREN ZUR REINIGUNG VON STEROLEN AUS TALLÖLPECH
PROCÉDÉ DE PURIFICATION DE STÉROLS À PARTIR DE BRAI DE TALL-OIL

(30) Priority: 01.12.2020 FI 20206236
(43) Date of publication of application: 11.10.2023
(73) Proprietor: Forchem Oyj, 26101 Rauma (FI)
(72) Inventor: ORTE, Juha, 26101 Rauma (FI); LINDEGREN, Henrik, 26101 Rauma (FI)
(74) Representative: Laine IP Oy
(86) International application number: PCT/FI2021/050834
(87) International publication number: WO 2022/117922

(56) References cited:
- WO-A1-2009/106696
- WO-A2-2008/099051

## Description

### FIELD OF THE INVENTION

The present invention relates to the production of sterols. The invention especially relates to the separation of the sterol neutral substances from tall oil pitch (TOP) by means of distillation or evaporation. The invention also relates to the production of fatty and/or resin acids from the residue obtained from the distillation or evaporation process.

### BACKGROUND OF THE INVENTION

Sterols are common name for a group of steroid alcohols having alcoholic OH group. In general, sterols present a chemical group consisting of a wide range of sterol compounds and isomers which can be found in plants. Industrially, sterols of various purities can be prepared from pure white fine powders to waxy sterol concentrated substances. Most common and known sterol compounds are cholesterol, sitosterol, and campesterol. Sterol compositions may vary depending on the source, e.g., human/animal species produce sterol compositions which are not found in plant-based sterols. Sterol composition may vary between different plants and is always containing some small quantities of more unusual sterols like brassicasterol and stigmasterol and some saturated sterols i.e. sitostanol and campestanol. Sterols are found and can be isolated from wide variety of plants, e.g., soy- sunflower-, maize-, corn, rice, and rapeseed but also from tree species. Tall oils may be among the most favorable raw material sources to obtain sterols. Most common industrial sources for tall oils are coniferous tree species like *Pinus Sylvesteris.* Major part of sterols occur as fatty acid esters thus these are concentrated into tall oil pitch (TOP) during crude tall oil (CTO) refining.

Crude tall oil (CTO) is obtained as a by-product of the kraft pulp process of coniferous trees. CTO is typically recovered from soap skimmed black liquor which contains high quantities of fatty acid sodium salts, resin acid sodium salts and unsaponifiables and neutral substances which include fatty alcohols and sterols.

In kraft pulp mills, skimmed soap is collected and acidulated with a mineral acid, such as sulphuric acid, to obtain an oil phase and a water phase. The oil phase, i.e. the crude tall oil, contains free fatty acids, resin acids and unsaponifiables where the amount of unsaponifiables can range from 10 to 35 w-% depending on the species and a quality of coniferous trees used. Crude tall oil is typically dried and distillated at high temperatures under vacuum to yield a light phase, a rosin phase, a fatty acid phase and a pitch phase.

The pitch phase, commonly known as tall oil pitch (TOP), is a black, tarry, heavy viscous distillation residue obtained from the first processing stages in crude tall oil refining. Tall oil pitch; EC 232-414-4; CAS 8016-81-7; is commonly used in many different products and applications such as pH regulation, water treatment products, extraction agents, polymers, lubricants and greases, coating products, fuels, adhesives and sealants and non-metal-surface treatment products and heavy fuels. In present time, tall oil pitch is a desirable product for biobased fuel manufacturing for many producers as such or in different biobased blends. The composition of the tall oil pitch is strongly dependent on the CTO raw material, distillation equipment and operating conditions employed in the distillation of tall oil. Thus, the composition may vary but in general it contains heaviest nonevaporable materials comprising resin acids, fatty acids and esters, polymeric materials, wax compounds, and large molecular weight materials and also unknown components. In all cases during the tall oil refining, pitch materials are removed and collected before tall oil fatty acids (TOFA). Pitch material contains concentrated amounts of sterols and steryl esters and these can be further purified.

Sterol extraction from tall oil pitch may be performed several ways after ester modification. Sterol fatty acid esters (steryl esters) are heavy and practically nonevaporable molecules which tend to decompose if processed in evaporable conditions over 320 °C. This is why steryl esters need to be modified to a smaller conformation before concentration with evaporative methods. Industrially, saponification is the easy, cheap, very efficient and also the most frequently used method to break down these difficultly collectable compounds before extraction. In saponification, ester bonds are deconstructed to free sterol forms and fatty acid soaps to obtain a processable sterol containing fraction.

A sterol containing fraction can be concentrated by using consecutive evaporations, distillations or combinations thereof. The isolation as well as concentration steps commonly comprise evaporational separations carried out in evaporation units, preferably by using technologies in art based on continuous film generating evaporators like Falling Film Evaporator (FFE), Wiped Film Evaporator (WFE), Thin Film Evaporator (TFE), or Short Path Evaporator (SPE) where free sterols are evaporated to the distillates and fatty acid soaps remain in residues. These concentration steps are performed under moderate or high vacuum and at sufficiently low temperatures to protect the sterols from degradation.

Highly concentrated sterol fractions can be produced with evaporators or distillation equipment or a combination thereof, such as a combination of thin film evaporator with adapted rectification column for the distillate.

Distillation/evaporation processes take advantage of the volatility and boiling point differences between volatile and non-volatile compounds in a chosen processing conditions dividing compounds into two or more phases. Evaporation is commonly performed by using high temperatures 180-310 °C with moderate or high vacuum being normally below 5 mbar. Distillation can be performed in many ways, but present industrial processes favor continuous tower columns. These columns are normally packed and distillation may be assisted by stripping gasses or superheated steam.

In the art of industrial engineering, the set-up or workflow for the evaporations and/or distillations can vary to achieve the same result. For example, to purify a sterol concentrated composition light evaporable compounds like free acids can be separated before sterols and heavy non-volatiles compounds can be separated in subsequent stages. All volatiles can also be separated from the non-volatiles at once and volatiles can be re-separated from each other in subsequent stages.

Concentrated sterols (Crude sterol) obtained form evaporation/distillation stages contain elevated amounts of free sterols. Sterol concentration may yield from >20 w-% up to 50 w-% or more and will also contain high amounts of light neutral materials with fatty and rosin acids. It is also known that some lighter, normally unevaporable materials can be conveyed into the lighter distillates due to powerful evaporation or material splashing in the evaporator, but small amounts of these may not spoil and terminate this industrial process. Crude sterols obtained as a distillate can be further purified. Widely used technologies involve the use of solvents where free sterols are isolated and purified by using solvent crystallization and filtration processes.

Distillation/evaporation processes which were described earlier take advantage of the volatility differences of volatile unsaponifiables, fatty acid, resin acid, and almost any other non-volatile materials such may be, e.g., organic acid soaps. In the case of separation by distillation, the difference between the boiling points of volatile products, such as unsaponifiable components, and the boiling point of different organic acid soaps is so remarkable that the separation is very efficient. However, a problem connected with this separation technique is the very high melting point and viscosity of the distillation residue, which leads to processing difficulties in handling the residue material, since removing and transferring material of high viscosity is very challenging.

US 6780831 discloses a method, wherein the problem described is solved by adding a high molecular weight softener to the saponified tall oil pitch prior to the evaporation of unsaponifiables. Examples of the non-evaporated softener are high molecular weight hydrocarbons, e.g. paraffin waxes, high boiling naphtenes, polyglycols, e.g. polypropylene glycol and polyethylene glycol, and high molecular weight silicon oils. These softeners lower the viscosity of the residual saponified pitch and thereby facilitate the evaporation process. However, in this method, the softeners can be environmentally harmful and removal of the softener from the final product may be required. Further, the transfer of the softerner back to the circulation incurs expenses because of extra processing.

US 8680324 discloses a process wherein a softener obtained only from the tall oil pitch is added to the saponified pitch. The softener is characterized by having pitchbased heavy components whitch are recirculated back to the dried saponified pitch in amounts 1-50% of the dried saponified pitch to enable evaporation of the unsaponifiable fraction during the first high vacuum evaporation. This method, however, is causing descending process capacity in the first evaporation step and the softening effect is only moderate after a few recycling steps. A process wherein sterols can be purified from tall oil pitch is disclosed in WO 2008/099051 A2.

Accordingly, there is still a need in the art for distillation/evaporation processes which would ensure high sterol yield from tall oil pitch and would provide easily processable hard pitch fractions which can be efficiently utilized, e.g. for biofuel production.

### SUMMARY OF THE INVENTION

It is an aim of the present invention to eliminate at least a part of the problems relating to the above technologies.

It is a further aim of the present invention to provide an effective processing method for preparing high yields of free sterols and a side stream of hard pitch fractions having lower melting point than in the prior art.

The present invention provides a process for purifying sterols from tall oil pitch, the method comprising the steps of:
a) saponifying tall oil pitch,
b) drying the saponified pitch,
c) subjecting the dried saponified pitch to a first evaporation fractionating to obtain an unsaponifiable, sterol rich, fraction as a distillate and a first residue,
d) optionally subjecting said distillate to a second evaporation fractionating to obtain an enriched unsaponifiable fraction and a second residue,
e) crystallizing sterols from the unsaponifiable fraction or the enriched unsaponifiable fraction in a solvent,
f) collecting the crystallized sterols from the solvent, thus producing a first residual fraction of the solvent,
g) washing or recrystallizing the collected crystallized sterols with a further volume of said solvent or with another solvent,
h) collecting the washed or recrystallized crystals from the solvent, thus producing a second residual fraction of the solvent,
i) regenerating said solvent from the first, second or any further residual fraction of the crystallization/washing solvent, a part thereof or a combination thereof to obtain regenerated solvent and residual dry matter;
j) recirculating at least part of the residual dry matter obtained from step i) to any of the steps a) to c) for use as a first softener, wherein the first softener is added to the tall oil pitch, the saponified tall oil pitch or the dried saponified tall oil pitch in at least one of the steps of a), b) and c), respectively;
k) adding a second softener to the first residue obtained in step c) and acidulating the mixture of said second softener and said first residue to obtain an aqueous phase and an organic phase, separating the aqueous phase and drying the organic phase to obtain a dried organic phase, wherein said second softener is an external softener selected from the group consisting of: a vegetable oil and a fatty acid residue obtained from tall oil refining process, and
l) optionally subjecting the dried organic phase to evaporation fractionating to obtain fatty and/or resin acids as a distillate and a bottom residue.

In the method, the second softener is an external softener selected from the group consisting of: a vegetable oil and a fatty acid residue obtained from tall oil refining process.

More specifically, the present processes are characterized by what is stated in claims 1-12.

Considerable advantages are obtained by the present invention. Effective processing of the raw-material, tall oil pitch, will allow for the recovery of free sterols together with hard pitch side stream fractions, which can be utilized for biofuel production. The present method can be used for the production of a number of products, for example one or more of food grade sterols having a purity of the sterols of at least 90 % by weight and hard pitch residue fractions having low melting point and improved viscosity for sufficient processing in further steps.

### BRIEF DESCRIPTION OF THE DRAWINGS

Figure 1 shows a simplified process scheme of a preferred embodiment of the invention.
Figure 2 shows a more detailed process scheme with sidestreams for recycling within the process.

### DESCRIPTION OF EMBODIMENTS

"Tall oil pitch" is a non-volatile fraction that is separated at crude tall oil vacuum distillation. Tall oil pitch (TOP) is the residue after distillation off (under vacuum) of a light phase, a rosin phase, and a fatty acid phase from crude tall oil.

The composition of the tall oil pitch varies depending on the origin of the tall oil and process. Typically, tall oil pitch contains some 30 to 60 % by weight of free acids, 20 to 40 % by weight of esterified acids, and 10 to 40 % by weight of unsaponifiable neutral compounds. More than 50 % by weight conventionally consists of high molecular components, about half of which are acidic components. The low molecular free acids are mainly dehydroabietic, abietic, and other resin acids. The esterified acids comprise primarily oleic and linoleic acids. Unsaponifiable fractions are composed of diterpene alcohols, fatty alcohols, sterols, and dehydrated sterols. The alcohol components are essentially present in esterified form.

Typically, in order to separate sterols from tall oil pitch, the process comprises a saponification step, which can be carried out by contacting TOP, the sterol rich fraction and/or hard pitch fraction thereof with an alkaline agent. In one embodiment, TOP is contacted with an alkaline agent selected from the group of alkali metal hydroxides, oxides and carbonates; earth alkaline metal hydroxides, oxides and carbonates, and combinations thereof. The alkaline agent is optionally used in the form of an aqueous solution or dispersion.

The alkaline agent can also be selected from alkali metal alkoxides, preferably in the form of a non-aqueous alcoholic solution or dispersion.

The amount of alkaline agent is selected depending on the extent of saponification to be achieved. Typically, saponification is carried out by contacting TOP, the sterol rich fraction or hard pitch fraction thereof with a stoichiometric excess of the alkaline agent.

Saponification can be carried out in a continuously operated reaction, for example in continuously stirred tank reactor (CSTR), such as a CSTR pressure reactor, or a tubular reactor. Saponification is typically continued until at least 75 mol %, in particular at least 85 %, suitably at least 95 mol % of the steryl esters have been saponified to yield the corresponding free sterols. The saponified fraction is dried to remove easily volatile components i.e. water or solvents due to saponification reagents. Softeners can be used to lower the viscosity of the residual saponified pitch and thereby facilitate the subsequent evaporation process.

The dried material is typically subjected to one or two steps of evaporation fractionating and the recovered crude sterols are purified by solvent crystallization.

In particular, the present invention is directed to a process for purifying sterols from tall oil pitch, the method comprising the steps of:
a) saponifying tall oil pitch, preferably in the presence of a first softener recirculated from solvent crystallization of sterols, wherein the saponification is preferably performed in the presence of an alkali hydroxide, more preferably sodium or potassium hydroxide or a mixture thereof, and wherein the amount of the first softener is preferably 7-20 weight %, more preferably 12-22 weight % and most preferably 15-25 weight % or more of the amount of total unsaponified feed without saponification reagents and water,
b) drying the saponified pitch,
c) subjecting the dried saponified pitch to a first evaporation fractionating to obtain an unsaponifiable, sterol rich fraction as a distillate and a first residue,
d) optionally subjecting said distillate to a second evaporation fractionating to obtain an enriched unsaponifiable fraction and a second residue,
e) crystallizing sterols from the unsaponifiable fraction or the enriched unsaponifiable fraction in a solvent,
f) collecting the crystallized sterols from the solvent, preferably by filtration, thus producing a first residual fraction of the solvent (and a concentrated fraction of crystallized sterols),
g) washing or recrystallizing the collected crystallized sterols with a further volume of said solvent or another solvent,
h) collecting the washed or recrystallized crystals from the solvent, preferably by filtration, thus producing a second residual fraction of the solvent (and a concentrated fraction of crystallized sterols),
i) regenerating said solvent from the first, second or any further residual fraction of the crystallization solvent, a part thereof or a combination thereof to obtain regenerated solvent and residual dry matter;
j) recirculating at least part of the residual dry matter obtained from step i) to any of the steps a) to c) for use as a first softener, wherein the first softener is added to the tall oil pitch, the saponified tall oil pitch or the dried saponified tall oil pitch in at least one of the steps of a), b) and c), respectively;
k) adding a second softener, preferably comprising a vegetable based waste oil stream or similar, e.g., extracted soybean oil and/or light distillation residues obtained from tall oil distillation i.e. distillation residue obtained from the fatty acid distillation, to the first residue obtained in step c) and acidulating the mixture of said second softener and said first residue to obtain an aqueous phase and an organic phase, separating the aqueous phase and drying the organic phase to obtain a dried organic phase, wherein said second softener is an external softener selected from the group consisting of: a vegetable oil and a fatty acid residue obtained from tall oil refining process, and
l) optionally subjecting the dried organic phase to evaporation fractionating to obtain fatty and/or resin acids as a distillate and a bottom residue.

In a preferred embodiment, the above method comprises a step of recirculating part of the dried organic phase obtained from steps k) or 1) into the saponification of step a) for use as a recirculated further softener.

In another preferred embodiment, in the step e), the crystallization is performed by dissolving the unsaponifiable fraction containing sterols in the crystallization solvent and crystallizing sterols by cooling the substance until sterols crystallize or by evaporating solvents until the rise of the sterol concentration forces crystallization.

In another preferred embodiment, the above method comprises an optional step of washing the collected crystallized sterols obtained from step h) with a further volume of said solvent. In another preferred embodiment, the above method comprises an optional step of recirculating residual solvent from any crystallization or washing step into the earlier crystallization or washing step without solvent regeneration.

In another preferred embodiment, the distillate rich in fatty acids and resin acids from step 1) is utilized in the preparation of biofuel. In preferred embodiments, the bottom fraction of heavy pitch obtained from step 1) and/or the dried organic phase obtained in step k) is/are utilized in the preparation of biofuel.

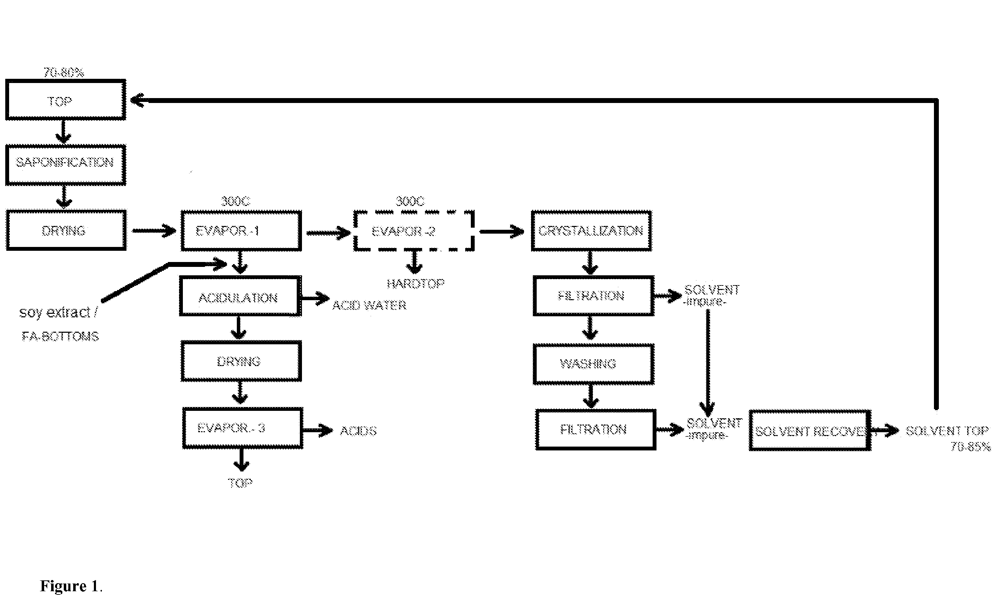

The present invention thus provides a method for purifying sterols from tall oil pitch in which at least two separate softeners are used. The first softener (i.e. saponification softener) is recirculated in a closed system of the present process as it is originating and recovered from the solvent used for crystallization of sterols and for washing the crystallized sterols (see Figure 1). In the step of saponification, the first softener is preferably added to the pitch feed or to the pitch soap. It is remarkable that the recovered solvent residue contains elevated quantities of sterols and therefore recirculation of the recovered solvent residue to saponification step may increase overall sterol yield.

In between the first evaporation step and the subsequent acidulation step performed to the first residue obtained from the first evaporation fractionating step, a second softener (i.e. residue softener), which is an external softener, i.e. not a product or fraction of the present process, is added to the first residue (see Figure 1). The second softener is added to the first residue at once after the evaporation process of step c) or while the first residue is separating in the evaporation process of step c). Preferably, the second softener should be of a type that would not excessively lower the vacuum level of the subsequent process steps so the second softener should be dry and degassed. Further, the second softener needs to be stabile when added in elevated temperatures that may be near 300 °C or above 200 °C and should also be miscible to the first residue and be able to maintain decreasing viscosity or free flowing properties and pumpability of the first residue. In present embodiments, the second softener can be selected from a group consisting of: residues from industrial treatments of peat or algae; vegetable fats, oils and fractions thereof; and animal fats, oils and fractions thereof. The second softener is preferably a bio waste component of animal, plant or soil origin that can be mixed with tall oil soap and is liquid and pumpable at the processing temperatures. More preferably, the second softener is a vegetable oil waste fraction, e.g., a soybean oil extract. Other possible fractions suitable for use as a second softener can be residues from industrial treatments of fats and oils, peat or algae or fractions from waxes or neutral substances of any other similar or suitable formed and recovered from the industrial or environmental processing i.e. fatty acid waste fractions, e.g. waste fractions formed in the separation of tall oils or wood oils, e.g. high neutral bottom fractions from separation columns, or waste fractions from refining of the above raw materials or fractions settling to the bottoms of storage tanks. In special embodiments, the second softener may contain waterless mineral acid, such as dehydrated 85 mol-% ortophosphoric acid, preferably so that the second softener contains 0.1 w-% - 3 w-% of said mineral acid. The amount of the mineral acid added to the second softener or directly to the saponified pitch residue should be adjusted in view of its effect to the vacuum level in the process.

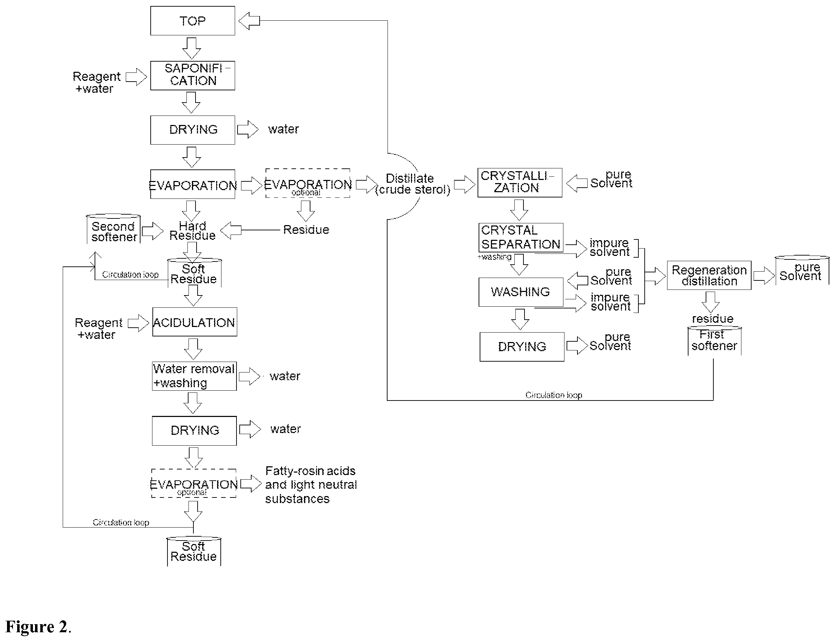

Accordingly, in a preferred embodiment, the mixing of the first residue and the second softener in step k) takes place as soon as possible after the evaporation fractionating or alternatively already during the evaporation step. In another preferred embodiment, part of the evaporation residue with added second softener is recycled and used as the second softener or mixed with the second softener in step k) (see the circulation loop shown in Figure 2 after first evaporation step). In another further preferred embodiment, the bottom residue obtained from step 1) is at least partly recirculated to step k) and is used as the second softener or mixed with the second softener (see Figure 2).

Preferably, the crystallization solvent used in any of steps e) to i) comprises hydrocarbon (such as ketone or ester), alcohol and optionally water, and most preferably the solvent comprises methyl ethyl ketone, methanol and water. The solvent used in steps e) and g) can be the same, or the solvent used for step e) may have a different composition than the solvent used in step g) or in any other further washing/re-crystallization step. Examples of processes for recovering sterols via crystallization are disclosed in WO00064922 and US7244856.

While the following examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

### EXPERIMENTAL SECTION

### Example 1. Saponification process without a softener.

The tall oil pitch (TOP) with high sterol content was saponified with aqueous NaOH, dried and then passed to an evaporation step performed at 300 °C and 2 mbar vacuum on a thin film evaporator in order to produce a distillate having a high sterol content. The melting point of saponified TOP was measured with a Mettler-Toledo melting point instrument.

### Results

Saponified TOP before the evaporation: melting point >200 °C
The bottom fraction obtained from the evaporation: melting point >200 °C

### Example 2. Saponification process with an added softener obtained from crystallization solvent

A light organic solvent fraction originating from the sterol crystallization purification step was metered into the tall oil pitch (TOP) in an amount of 20-25 weight % of the total amount of TOP. The mixture obtained was saponified with aqueous NaOH, dried and then passed to an evaporation step performed at 300 °C and 2 mbar vacuum on a thin film evaporator. The evaporation residue was blended at 180-230 °C with a 1:1.5 mixture of vegetable oil and a light residue from the tall oil refinery. The melting points of the obtained products were measured with a Mettler-Toledo melting point instrument.

### Results

Saponified TOP with added solvent fraction (before the evaporation): melting point at 125 °C
The evaporation residue before addition of the mixture of vegetable/wood oil: melting point at 142 °C
The evaporation residue after addition of the mixture of vegetable/wood oil: melting point at 128 °C

### Example 3. Saponification process with an added softener according to prior art

The tall oil pitch (TOP) with high sterol content was saponified with aqueous NaOH and dried. Then acid neutralized, washed and dried heavy pitch in an amount of 50 weight % of the total amount of the dried soap was added to the dried soap and the mixture was subjected to an evaporation step performed at 300 °C and 2 mbar vacuum on a thin film evaporator. The melting points of the obtained products were measured with a Mettler-Toledo melting point instrument.

### Results

Saponified TOP with added heavy pitch fraction before evaporation: melting point at 141 °C
The evaporation bottom residue after evaporation: melting point at 196 °C

### Conclusion

The present method (of Example 2) provides easily processable hard pitch fractions having lower melting point and also improved viscosity than in the prior art (Example 1 and 3).

### REFERENCES

Cited patent literature: US6780831, US8680324, US7244856 and WO0064922

## Claims

1. Process for purifying sterols from tall oil pitch, the method comprising the steps of:
a) saponifying tall oil pitch,
b) drying the saponified pitch,
c) subjecting the dried saponified pitch to a first evaporation fractionating to obtain an unsaponifiable, sterol rich fraction as a distillate and a first residue,
d) optionally subjecting said distillate to a second evaporation fractionating to obtain an enriched unsaponifiable fraction and a second residue,
e) crystallizing sterols from the unsaponifiable fraction or the enriched unsaponifiable fraction in a solvent,
f) collecting the crystallized sterols from the solvent, thus producing a first residual fraction of the solvent,
g) washing or recrystallizing the collected crystallized sterols with a further volume of said solvent or with another solvent,
h) collecting the washed or recrystallized crystals from the solvent, thus producing a second residual fraction of the solvent,
i) regenerating said solvent from the first, second or any further residual fraction of the crystallization solvent, a part thereof or a combination thereof to obtain regenerated solvent and residual dry matter;
j) recirculating at least part of the residual dry matter obtained from step i) to any of the steps a) to c) for use as a first softener, wherein the first softener is added to the tall oil pitch, the saponified tall oil pitch or the dried saponified tall oil pitch in at least one of the steps of a), b) and c), respectively;
k) adding a second softener to the first residue obtained in step c) and acidulating the mixture of said second softener and said first residue to obtain an aqueous phase and an organic phase, separating the aqueous phase and drying the organic phase to obtain a dried organic phase, wherein said second softener is an external softener selected from the group consisting of: a vegetable oil and a fatty acid residue obtained from tall oil refining process, and
l) optionally subjecting the dried organic phase to evaporation fractionating to obtain fatty and/or resin acids as a distillate and a bottom residue.

2. The process according to claim 1, wherein the evaporation fractionation of step c) is carried out in a thin film-, or short path evaporator with or without adapted rectification equipment.

3. The process according to claim 2, wherein the thin film evaporator operates at a temperature in the range of 270°C to 310°C, preferably 290°C to 300°C and at a pressure in the range of 10 to 500 Pa, preferably 100 to 200 Pa.

4. The process according to any one of claims 1-3, wherein the solvent of step e) comprises hydrocarbon such as an ester or ketone, alcohol and optionally water, and most preferably the solvent comprises methyl ethyl ketone, methanol and water.

5. The process according to any one of claims 1-4, wherein the saponification step a) is performed in the presence of NaOH and/or KOH.

6. The process according to any one of claims 1 to 5, wherein the distillate rich in fatty acids and resin acids from step l) is utilized in the preparation of biofuel.

7. The process according to any one of claims 1 to 5, wherein the bottom fraction of heavy pitch obtained from step l) is utilized in the preparation of biofuel and/or wherein the dried organic phase obtained in step k) is utilized in the preparation of biofuel.

8. The process according to any one of claims 1-6, wherein said vegetable oil is a soybean oil extract.

9. The process according to any one of claims 1-6, wherein said fatty acid residue obtained from tall oil refining process is the fatty acid residue obtained from the tall oil fatty acid (TOFA) or tall oil rosin (TOR) recovering stages comprising increased amounts of resin acids, fatty acid esters, polymers and dimerized substances.

10. The process according to any one of claims 1-9, wherein the regenerating step i) is performed as an evaporation fractionating method.

11. The process according to any one of claims 1-10, wherein the collecting step f) and/or h) is/are performed by filtrating the crystallized sterols from the solvent.

12. The process according to any one of claims 1-11, wherein part of the first residue with added second softener is recycled in step k) and is used as the second softener or is mixed with the second softener.

## Patentansprüche

1. Prozess zur Reinigung von Sterolen aus Tallölpech, wobei das Verfahren die Schritte umfasst zum:
a) Verseifen von Tallölpech,
b) Trocknen des verseiften Pechs,
c) Unterziehen des getrockneten verseiften Pechs einer ersten Verdampfungsfraktionierung, um eine unverseifbare, sterolreiche Fraktion als Destillat und einen ersten Rückstand zu erhalten,
d) optional Unterziehen des Destillats einer zweiten Verdampfungsfraktionierung, um eine angereicherte unverseifbare Fraktion und einen zweiten Rückstand zu erhalten,
e) Kristallisieren von Sterolen aus der unverseifbaren Fraktion oder der angereicherten unverseifbaren Fraktion in einem Lösungsmittel,
f) Sammeln der kristallisierten Sterole aus dem Lösungsmittel, wodurch eine erste Restfraktion des Lösungsmittels erzeugt wird,
g) Waschen oder Umkristallisieren der gesammelten kristallisierten Sterole mit einem weiteren Volumen des Lösungsmittels oder mit einem anderen Lösungsmittel,
h) Sammeln der gewaschenen oder umkristallisierten Kristalle aus dem Lösungsmittel, wodurch eine zweite Restfraktion des Lösungsmittels erzeugt wird,
i) Regenerieren des Lösungsmittels aus der ersten, zweiten oder beliebigen weiteren Restfraktion des Kristallisationslösungsmittels, einem Teil davon oder einer Kombination davon, um regeneriertes Lösungsmittel und Resttrockenmasse zu erhalten;
j) Rückführen mindestens eines Teils der aus Schritt i) erhaltenen Resttrockenmasse in einen der Schritte a) bis c) zur Verwendung als erster Weichmacher, wobei der erste Weichmacher dem Tallölpech, dem verseiften Tallölpech oder dem getrockneten verseiften Tallölpech in mindestens einem der Schritte a), b) und c) zugesetzt wird;
k) Zusetzen eines zweiten Weichmachers zu dem in Schritt c) erhaltenen ersten Rückstand und Ansäuern der Mischung aus dem zweiten Weichmacher und dem ersten Rückstand, um eine wässrige Phase und eine organische Phase zu erhalten, Abscheiden der wässrigen Phase und Trocknen der organischen Phase, um eine getrocknete organische Phase zu erhalten, wobei der zweite Weichmacher ein externer Weichmacher ist, ausgewählt aus der Gruppe bestehend aus: einem Pflanzenöl und einem Fettsäurerückstand, der aus dem Tallöl-Raffinationsprozess erhalten wird, und
l) optional Unterziehen der getrockneten organischen Phase einer Verdampfungsfraktionierung, um Fett- und/oder Harzsäuren als Destillat und Bodenrückstand zu erhalten.

2. Prozess nach Anspruch 1, wobei die Verdampfungsfraktionierung in Schritt c) in einem Dünnschicht- oder Kurzwegverdampfer mit oder ohne angepasster Rektifikationsausrüstung ausgeführt wird.

3. Prozess nach Anspruch 2, wobei der Dünnschichtverdampfer bei einer Temperatur im Bereich von 270°C bis 310°C, vorzugsweise 290°C bis 300°C und mit einem Druck im Bereich von 10 bis 500 Pa, vorzugsweise 100 bis 200 Pa, arbeitet.

4. Prozess nach einem der Ansprüche 1-3, wobei das Lösungsmittel in Schritt e) Kohlenwasserstoff wie etwa einen Ester oder Keton, Alkohol und optional Wasser umfasst und das Lösungsmittel besonders bevorzugt Methylethylketon, Methanol und Wasser umfasst.

5. Prozess nach einem der Ansprüche 1-4, wobei der Verseifungsschritt a) in Anwesenheit von NaOH und/oder KOH durchgeführt wird.

6. Prozess nach einem der Ansprüche 1 bis 5, wobei das an Fettsäuren und Harzsäuren reiche Destillat aus Schritt 1) zur Herstellung von Biokraftstoff verwendet wird.

7. Prozess nach einem der Ansprüche 1 bis 5, wobei die aus Schritt 1) erhaltene Bodenfraktion von schwerem Pech bei der Herstellung von Biokraftstoff verwendet wird, und/oder wobei die in Schritt k) erhaltene getrocknete organische Phase bei der Herstellung von Biokraftstoff verwendet wird.

8. Prozess nach einem der Ansprüche 1-6, wobei das Pflanzenöl ein Sojabohnenölextrakt ist.

9. Prozess nach einem der Ansprüche 1-6, wobei der aus dem Tallöl-Raffinationsprozess erhaltene Fettsäurerückstand der aus den Rückgewinnungsstufen der Tallölfettsäure (TOFA) oder Tallölharz (TOR) erhaltene Fettsäurerückstand ist, der erhöhte Mengen an Harzsäuren, Fettsäureestern, Polymeren und dimerisierten Substanzen umfasst.

10. Prozess nach einem der Ansprüche 1-9, wobei der Regenerationsschritt i) als ein Verdampfungsfraktionierungsverfahren durchgeführt wird.

11. Prozess nach einem der Ansprüche 1-10, wobei der Sammelschritt f) und/oder h) durch Filtrieren der kristallisierten Sterole aus dem Lösungsmittel durchgeführt wird/werden.

12. Prozess nach einem der Ansprüche 1-11, wobei ein Teil des ersten Rückstands mit zugesetztem zweiten Weichmacher in Schritt k) recycelt und als zweiter Weichmacher verwendet oder mit dem zweiten Weichmacher vermischt wird.

## Revendications

1. Processus de purification de stérols à partir de brai de tall oil, le procédé comprenant les étapes consistant à :
a) saponifier du brai d'huile de tall,
b) sécher le brai saponifié,
c) soumettre le brai saponifié séché à un premier fractionnement par évaporation pour obtenir une fraction insaponifiable riche en stérols sous la forme d'un distillat et un premier résidu,
d) soumettre facultativement ledit distillat à un second fractionnement par évaporation pour obtenir une fraction insaponifiable enrichie et un second résidu,
e) cristalliser les stérols à partir de la fraction insaponifiable ou de la fraction insaponifiable enrichie dans un solvant,
f) collecter les stérols cristallisés à partir du solvant, produisant ainsi une première fraction résiduelle du solvant,
g) laver ou recristalliser les stérols cristallisés collectés avec un volume supplémentaire dudit solvant ou avec un autre solvant,
h) collecter les cristaux lavés ou recristallisés à partir du solvant, produisant ainsi une seconde fraction résiduelle du solvant,
i) régénérer ledit solvant à partir de la première, de la seconde ou de toute fraction résiduelle supplémentaire du solvant de cristallisation, d'une partie de celle-ci ou d'une combinaison de celles-ci pour obtenir un solvant régénéré et une matière sèche résiduelle ;
j) remettre en circulation au moins une partie de la matière sèche résiduelle obtenue à l'étape i) vers l'une quelconque des étapes a) à c) pour l'utiliser comme premier adoucissant, dans lequel le premier adoucissant est ajouté au brai d'huile de tall, au brai d'huile de tall saponifié ou au brai d'huile de tall saponifié séché dans au moins l'une des étapes a), b) et c), respectivement ;
k) ajouter un second adoucissant au premier résidu obtenu à l'étape c) et aciduler le mélange dudit second adoucissant et dudit premier résidu pour obtenir une phase aqueuse et une phase organique, séparer la phase aqueuse et sécher la phase organique pour obtenir une phase organique séchée, dans lequel ledit second adoucissant est un adoucissant externe choisi dans le groupe consistant en : une huile végétale et un résidu d'acides gras obtenu à partir du processus de raffinage d'huile de tall, et
l) soumettre facultativement la phase organique séchée à un fractionnement par évaporation pour obtenir des acides gras et/ou résiniques sous la forme d'un distillat et un résidu de fond.

2. Processus selon la revendication 1, dans lequel le fractionnement par évaporation de l'étape c) est effectué dans un évaporateur à couche mince ou à trajet court avec ou sans équipement de rectification adapté.

3. Processus selon la revendication 2, dans lequel l'évaporateur à couche mince fonctionne à une température dans la plage allant de 270 °C à 310 °C, de préférence de 290 °C à 300 °C et à une pression dans la plage allant de 10 à 500 Pa, de préférence de 100 à 200 Pa.

4. Processus selon l'une quelconque des revendications 1-3, dans lequel le solvant de l'étape e) comprend un hydrocarbure tel qu'un ester ou une cétone, un alcool et facultativement de l'eau, et le plus préférentiellement le solvant comprend de la méthyléthylcétone, du méthanol et de l'eau.

5. Processus selon l'une quelconque des revendications 1-4, dans lequel l'étape de saponification a) est réalisée en présence de NaOH et/ou KOH.

6. Processus selon l'une quelconque des revendications 1 à 5, dans lequel le distillat riche en acides gras et en acides résiniques de l'étape l) est utilisé dans la préparation de biocarburant.

7. Processus selon l'une quelconque des revendications 1 à 5, dans lequel la fraction de fond de brai lourd obtenue à l'étape l) est utilisée dans la préparation de biocarburant et/ou dans lequel la phase organique séchée obtenue à l'étape k) est utilisée dans la préparation de biocarburant.

8. Processus selon l'une quelconque des revendications 1-6, dans lequel ladite huile végétale est un extrait d'huile de soja.

9. Processus selon l'une quelconque des revendications 1-6, dans lequel ledit résidu d'acides gras obtenu à partir du processus de raffinage d'huile de tall est le résidu d'acides gras obtenu à partir des étapes de récupération d'acides gras d'huile de tall (TOFA) ou de colophane d'huile de tall (TOR) comprenant des quantités accrues d'acides résiniques, d'esters d'acides gras, de polymères et de substances dimérisées.

10. Processus selon l'une quelconque des revendications 1-9, dans lequel l'étape de régénération i) est réalisée sous la forme d'un procédé de fractionnement par évaporation.

11. Processus selon l'une quelconque des revendications 1-10, dans lequel l'étape de collecte f) et/ou h) est/sont réalisée(s) en filtrant les stérols cristallisés à partir du solvant.

12. Processus selon l'une quelconque des revendications 1-11, dans lequel une partie du premier résidu avec un second adoucissant ajouté est recyclée à l'étape k) et est utilisée comme second adoucissant ou est mélangée avec le second adoucissant.
